# FASCICULE DE BREVET EUROPEEN

(11) **EP 2 464 713 B1**
(45) Date de publication et mention de la délivrance du brevet: **07.05.2014**
(21) Numéro de dépôt: 10761035.4
(22) Date de dépôt: 10.08.2010
(51) Int. Cl.: C11B 1/00, A61K 36/63, C11B 1/06, A23L 1/05

(54) **PROCEDE DE PREPARATION D'UN JUS INTEGRAL D'OLIVE, COMPOSITION OBTENUE SELON CE PROCEDE ET SON APPLICATION DANS LE DOMAINE DE LA COSMETIQUE ET DE LA DIETETIQUE**
VERFAHREN ZUR HERSTELLUNG EINES VOLLWERTIGEN SAFTES AUS OLIVEN, DADURCH HERGESTELLTE ZUBEREITUNG UND DEREN VERWENDUNG IM BEREICH DER KOSMETIK UND DER ERNÄHRUNG
PROCESS FOR THE PREPARATION OF AN INTEGRAL OLIVE JUICE, COMPOSITION SO OBTAINED, AND ITS USE IN COSMETIC AND IN DIETETIC FIELDS

(30) Priorité: 11.08.2009 FR 0955618
(43) Date de publication de la demande: 20.06.2012
(73) Titulaire: Totum Bio Cosmetics, 34120 Pézenas (FR)
(72) Inventeur: COLICCI, Raphaël, F-34700 Saint-Privat (FR); DUBOIS, Jacques, F-11100 Narbonne (FR); LAFAURIE, Jean-Louis, F-34600 Herepian (FR)
(74) Mandataire: Hartmann, Jean-Luc
(86) Numéro de dépôt international: PCT/FR2010/051688
(87) Numéro de publication internationale: WO 2011/018579

(56) Documents cités:
- EP-A1- 1 211 303
- WO-A1-00/04794
- FR-A1- 2 841 481
- FR-A1- 2 867 071
- US-A- 4 370 274

## Description

La présente invention a pour objet un procédé de préparation d'un jus intégral d'olive ainsi qu'une composition à base de jus intégral d'olive obtenue selon ce procédé. L'utilisation de cette composition dans le domaine de la cosmétique et de la diététique est également un objet de la présente invention.

L'olivier (Olea europea) et ses produits dérivés sont connus depuis des millénaires. On retrouve des traces de feuilles fossilisées sur le site de Santorin datant de 37 000 ans av. J-C, des traces de charbon et de pollen d'olivier datant de 12 000 ans av. J-C ont été identifiées en bordure du Sahara.

L'olive est une drupe à noyau dur poussant sur des arbres parfois centenaires et même millénaires en Palestine, Grèce et Sicile. De nombreuses variétés d'oliviers sont cultivées autour de la Méditerranée mais aussi en Californie, Australie, Afrique du Sud et Chine. Les olives vertes sont cueillies avant la maturation complète du fruit (en septembre pour le bassin méditerranéen), alors que la couleur noire ne se développe qu'à complète maturité (de décembre à février pour le bassin méditerranéen).

En raison de l'amertume apportée par l'hétéroglucoside principal (Oleuropéine) qu'elles contiennent dans la pulpe, les olives sont impropres à la consommation tant qu'elles ne sont pas traitées par une solution alcaline qui provoque l'hydrolyse de ce glucoside amer ; après rinçage à l'eau elles sont conservées dans de la saumure.

L'huile est obtenue selon divers procédés bien connus, les olives destinées à cette production sont lavées, puis broyées pour former une pâte ; après malaxage cette pâte est pressurée puis décantée ou centrifugée pour séparer les éléments solides, ou « grignons », de la phase liquide composée d'huile et de solution aqueuse appelées « margines ». Le rendement en huile varie avec l'espèce, le procédé d'extraction et le degré de maturité de l'olive. Ainsi, avec des olives parfaitement matures, il est fréquent d'obtenir 12 à 25 kg d'huile pour 100 kg de fruits.

Il faut noter que ce rendement est bien plus faible avec des olives récoltées avant maturité et s'abaisse à 5-6 % d'huile dans le cas de récoltes précoces. Traditionnellement cette huile de couleur et de composition particulière dénommée « omphacine » était utilisée par les parfumeurs grecs puis par les Romains en onctions corporelles.

Très tôt les hommes ont su utiliser l'olive et ses produits dérivés de différentes façons. À des fins alimentaires, les fruits verts ou noirs se sont prêtés à une grande diversité de préparations et constituent des spécialités locales fort appréciées.

Par ailleurs, l'huile obtenue de préférence par pression à froid (huile vierge extra) constitue l'élément essentiel du régime crétois et plus largement méditerranéen. Le paradoxe de ce régime, actuellement bien compris, est lié à la composition des triglycérides dont 75 % incluent un acide gras monoinsaturé : l'acide oléique. De plus, on a relevé la présence de composés phénoliques : hétéroglucosides (oleuropéine, ligstroside ou oléocanthal) et les phénols dérivés de leur hydrolyse (tyrosol, hydroxytyrosol). Il faut noter que l'huile d'olive contient en outre des vitamines (notamment A et E), du squalane et des stérols dans la fraction insaponifiable.

A côté de ces applications alimentaires, l'huile est utilisée dans le domaine pharmaceutique par voie orale ou en lavement en raison de ses propriétés cholaguogues, légèrement laxatives.

En usage externe, ses propriétés adoucissantes et émollientes justifient son emploi dermatologique (liniment oléo-calcaire notamment) ; elle sert de véhicule dans diverses préparations du Codex : huile camphrée ou huile à l'essence de niaouli par exemple. Elle est utilisée comme solvant de certaines préparations injectables après neutralisation et stérilisation dans les conditions définies par la pharmacopée.

Dans le domaine industriel, l'huile d'olive donne lieu à d'importantes applications, telles que la fabrication de savons ou la préparation de produits cosmétologiques.

Les tourteaux ou « grignons », résidus solides séparés après malaxage des drupes sont récupérés et peuvent servir pour l'alimentation du bétail ou éventuellement comme engrais.

Enfin, les margines, obtenues après séparation systématique de l'huile de la phase liquide, constituent un effluent riche en matières organiques et créent un problème réel de pollution à l'industrie oléicole, en l'absence de toute valorisation de ses composants.

Actuellement, depuis la reconnaissance de l'intérêt du régime crétois, un élan nouveau s'est développé en faveur de l'utilisation de l'olive à des fins diététiques et thérapeutiques. Récemment, la recherche scientifique a mis en évidence les effets des polyphénols de l'huile d'olive : action antioxydante et augmentation des taux de HDL cholestérol, expliquant ainsi le ralentissement des phénomènes liés au vieillissement et la prévention de certaines maladies : affections cardio-vasculaires, ostéoporose, formation de tumeurs.

La présente invention vise à exploiter les propriétés bien connues de l'huile résultant de sa composition (triglycérides d'acide gras mono-insaturés, hétéroglucosides, vitamines A et E, squalane, stérols, composés phénoliques) et celles non utilisées jusqu'à ce jour des margines riches en actifs d'intérêt, notamment sels minéraux (Ca, Mg, P, K) et polyphénols à concentration élevée. Ces derniers constituants sont pourtant constamment éliminés pour l'obtention de l'huile qui est utilisée isolément pour ses propriétés intrinsèques. Ces margines constituent ainsi un effluent encombrant, d'autant plus polluant que les techniques modernes d'extraction industrielle en continu entraînent une forte augmentation de leur volume en raison de l'addition de suppléments d'eau lors des centrifugations afin d'augmenter le rendement de l'extraction d'huile.

Le procédé traditionnel d'obtention de l'huile, quant à lui met en oeuvre une méthode discontinue comprenant les étapes de broyage, malaxage puis pression et enfin décantation et centrifugation, sans addition d'eau. La phase aqueuse (margines) est ensuite systématiquement séparée de la phase huileuse qui est considérée comme le produit noble du fruit. Selon ce procédé traditionnel, les margines sont plus concentrées (teneur en extrait sec environ deux fois plus élevée) que celles obtenues par procédé industriel et constituent de ce fait un déchet d'autant plus polluant qu'il est resté longtemps considéré sans intérêt.

Des essais de valorisation de ces margines ont été menés afin de réduire le caractère polluant de ce résidu et de tirer partie de ses constituants, mais sans grand succès à ce jour. La difficulté tient à l'instabilité des polyphénols qui s'oxydent rapidement, rendant la conservation de cette phase aqueuse problématique. Certains ont cru résoudre ce problème en atomisant ou évaporant cette solution afin d'obtenir une poudre stable, plus facilement manipulable. Mais cette transformation entraîne par elle-même une rapide dégradation des composés qu'elle vise à stabiliser.

Les bénéfices résultant de l'association des actifs stabilisés des margines avec l'huile d'olive n'ont jamais été concrétisés. FR 2867071 décrit une composition associant un extrait sec de margines avec de l'huile d'olive peroxydée par ozonisation pour conférer de nouvelles propriétés à cette phase. Le produit obtenu fait ainsi l'objet de manipulations complexes et coûteuses, alourdissant le procédé de fabrication et aboutissant de surcroît à un produit transformé qui ne répond plus aux caractéristiques naturelles de l'huile d'olive et qui n'en présente plus les mêmes qualités.

La présente invention apporte une solution à ces problèmes. S'inspirant de la méthode ancestrale discontinue, les inventeurs ont mis au point un procédé permettant d'obtenir directement sans séparation des phases aqueuses et huileuses le jus intégral de l'olive, après pression de la pâte et séparation des résidus solides tout en obtenant une bonne stabilisation des actifs de la phase aqueuse.

Plus précisément, la présente invention consiste en un procédé de préparation d'un jus intégral d'olive caractérisé en ce qu'il comprend les étapes suivantes :
a) sélectionner des olives en fonction de leur maturité,
b) broyer et malaxer les olives choisies sous forme de drupes,
c) presser la pâte obtenue à l'étape précédente,
d) séparer le jus obtenu pour le dissocier des éléments solides,
e) recueillir le jus intégral d'olive sous forme d'une émulsion gélifiée stable.

Par opposition à la méthode ancestrale discontinue dont le but est de séparer les deux phases pour mieux exploiter les propriétés intrinsèques de l'huile d'olive et se débarrasser par la même occasion des margines considérées comme néfastes, le procédé selon l'invention vise précisément à maintenir en contact intime les deux phases. De manière surprenante, les inventeurs ont en effet constaté, outre l'avantage en terme de simplicité et de temps de préparation, que la qualité et les propriétés de la phase huileuse ne sont pas altérées par la présence des margines. Au contraire, les bénéfices de l'huile d'olive sont potentialisés par cette association grâce aux constituants et actifs d'intérêt de la phase aqueuse. Ces actifs se révèlent d'autant plus bénéfiques qu'ils se présentent sous une forme native, non transformés par une étape de traitement supplémentaire. Le contact avec la phase huileuse semble également jouer un rôle protecteur et stabilisant vis à vis des constituants des margines dont les propriétés sont maintenues optimales.

Le jus intégral d'olive obtenu à l'issu de ce procédé se présente sous la forme d'une émulsion gélifiée naturellement stabilisée par les interactions spontanées de la phase huileuse avec la phase aqueuse. Pour augmenter la durée de conservation et de stabilité de ce jus intégral, il est possible d'y incorporer immédiatement après l'étape e) des agents viscosants et éventuellement émulgateurs ou un mélange de ceux-ci.

Pour optimiser la qualité du jus intégral d'olive, le procédé objet de l'invention est réalisé de préférence à froid et à l'abri de l'air.

Au cours des travaux de réalisation et de mise au point de ce nouveau procédé, les inventeurs ont été conduits à utiliser des olives obtenues pendant toute la durée de récolte possible allant d'un état de maturité précoce à avancé (respectivement de septembre à janvier-février pour le bassin méditerranéen). Ce hasard leur a permis de découvrir l'existence de variations importantes de composition entre les produits obtenus à partir d'olives matures et ceux obtenus à partir de fruits cueillis le plus précocement possible, semblables à ceux qui étaient traités dans l'antiquité pour obtenir l'huile omphacine. Ces variations se traduisent notamment en terme de couleur, d'activité vitaminique, de quantité d'acide oléique et de biophénols, ainsi qu'en terme de rendement en jus et en huile. Ces données ont été mesurées et figurent dans les exemples de la présente demande.

Ainsi, le procédé selon la présente invention peut comprendre à l'étape a) la sélection d'olives majoritairement récoltées à un stade précoce de maturité (caractérisé par des olives de couleur verte, des drupes fermes et lisses, riches en eau) ce qui permet de bénéficier de toutes les propriétés avantageuses liées à l'omphacine. Dans ce cas, de préférence, l'étape a) comprend la sélection d'olives uniquement récoltées à un stade précoce de maturité.

De manière alternative, le procédé selon la présente invention peut également comprendre à l'étape a) la sélection d'olives majoritairement récoltées à un stade de maturité avancé (caractérisé par des olives de couleur noir en raison d'une plus forte teneur en verbascoside, des drupes mures et frippées, dont la teneur en eau est plus faible et le contenu huileux est plus élevé) afin de bénéficier des propriétés avantageuses d'une huile plus mature. Dans ce cas, de préférence, l'étape a) comprend la sélection d'olives uniquement récoltées à un stade de maturité avancé.

La présente invention concerne également une composition à base de jus intégral d'olives obtenu selon le procédé décrit ci-dessus, caractérisée en ce qu'elle comprend un mélange de phase huileuse et de phase aqueuse d'olive non altérées par des étapes agressives de traitement. Ce suc intégral d'olive contient en effet tous les ingrédients de l'olive à la plus haute concentration possible et sous la forme la plus naturelle possible ; ils font l'intérêt du régime crétois (glycérides oléiques, composés phénoliques, vitamines A et E, stérols, squalanes,...) dans la perspective d'utilisations alimentaires diététiques. Il apporte aussi toutes les qualités requises pour servir de base à des préparations cosmétiques.

Selon le moment où les fruits sont cueillis, le jus intégral d'olive se présente sous la forme d'un mélange constitué d'une huile en proportion variable entre 5 et 25 % en poids et d'une solution aqueuse comprise entre 95 et 75 % en poids. La composition selon l'invention comprend ainsi de préférence 5 à 25 % en poids de ladite composition de phase huileuse et 95 à 75 % en poids de ladite composition de phase aqueuse.

La possibilité d'ajuster le pourcentage relatif de la phase huileuse, par addition d'huile vierge obtenue indépendamment du procédé revendiqué n'est pas exclue, ceci afin d'ajuster la proportion d'huile présente dans le produit fini en fonction de son application. Cet ajout d'huile permet de renforcer le caractère nourrissant et protecteur d'une crème de nuit par exemple. Il n'est pas nécessaire si l'on souhaite obtenir une émulsion de texture plus légère telle qu'un lait hydratant.

Pour faciliter son utilisation, et sa conservation, ce nouveau produit peut être stabilisé, par l'utilisation de divers additifs répondants de préférence aux exigences des labels bio (tel qu'ECOCERT, par exemple, pour la France). Dans un mode particulier de réalisation, la composition selon l'invention peut comprendre en outre des additifs choisis parmi les gélifiants, les antioxydants hydrosolubles, conservateurs, chélateurs ou un mélange de ceux-ci.

L'addition de gélifiants, par exemple, permet d'augmenter la viscosité et maintenir le mélange homogène en évitant la séparation des deux phases. A titre indicatif et non limitatif, nous citerons les gélifiants classiques, en retenant ceux qui sont recommandés dans le domaine alimentaire : alginates, carraghenanes, pectines, gomme arabique, gomme adragante et plus particulièrement gomme guar et gomme xanthane. Leur concentration varie selon le produit entre 0,1 et 3 % en poids.

L'addition d'antioxydants hydrosolubles, de préférence naturel tel que l'acide ascorbique peut être réalisée.

La protection microbiologique peut être obtenue par un procédé physique tel que le traitement UHT classique pour le lait ou par addition d'agents conservateurs autorisés : acide salicylique, acide benzoïque, acide sorbique ainsi que leurs sels ou leur association. La concentration est comprise entre 0,05 et 1 % en poids.

Des chélateurs peuvent également être incorporés dans la composition finale.

De manière surprenante, les inventeurs ont imaginé utiliser la composition obtenue selon le procédé objet de l'invention, sans autre traitement, dans le domaine de la cosmétique et/ou de la diététique.

Les applications envisagées se prêtent en effet à l'utilisation directe de ce jus intégral, en s'exonérant de l'étape de séparation des phases aqueuses et huileuses. Le produit obtenu se révèle particulièrement avantageux en ce qu'il contient la totalité des constituants liquides, solubles dans l'eau et dans l'huile, sans perte d'actifs à l'état natif, sans risque d'altération, avec une économie de manipulations, en réduisant les dépenses d'énergies et en évitant l'accumulation d'effluents embarrassants dans une préoccupation écologique.

De préférence, l'utilisation de la composition obtenue selon le procédé objet de l'invention est réalisée dans le domaine de la cosmétique dans le cas d'une composition élaborée avec une majorité d'olives récoltées à un stade précoce de maturité. Cette utilisation se révèle en effet particulièrement avantageuse grâce à une action anti-radicalaire, anti-inflammatoire non thérapeutique, émolliente et adoucissante de cette composition.

De manière alternative, l'utilisation de la composition obtenue selon le procédé objet de l'invention est réalisée dans le domaine de la diététique dans le cas d'une composition élaborée avec une majorité d'olives récoltées à un stade de maturité avancé. Cette utilisation se révèle en effet particulièrement avantageuse grâce à une action anti-radicalaire, de prévention des affections cardio-vasculaires et des lésions tumorales de cette composition.

Ainsi préparé, ce nouveau produit est conditionné dans des récipients étanches et hermétiques à l'air et stocké à l'abri de la lumière et de la chaleur parfaitement remplis, l'air restant pouvant être remplacé par du gaz carbonique ou de l'azote ; leur volume est adapté à celui de l'unité opératoire finale.

Les exemples qui suivent illustrent l'invention afin d'en faire mieux apparaître les caractéristiques et les avantages, sans toutefois en réduire de quelque manière que ce soit la portée.

### EXEMPLES

Pour illustrer les différences de composition entre les olives précoces et les olives matures, certaines caractéristiques ont été mesurées avec des olives de variété « Picholine » et des olives de variété « Lucques » récoltées en septembre (omphacine) ou en décembre (huile classique) 2008.
- couleur :
Le spectre d'absorption révèle un pic à 420 nm dans l'omphacine et l'huile classique;
un deuxième pic à 670 nm est observé dans l'omphacine seule.
- activité vitaminique E :
- 36 mg pour 100 grammes de matière grasse (g.MG) d'omphacine Picholine,
   contre 17 mg/100 g.MG d'huile classique Picholine.
- 14,5 mg/100 g.MG d'omphacine Lucques,
contre 8,1 mg/100 g.MG d'huile classique Lucques.
Soit une teneur en tocotrienol environ 2 fois plus élevée dans l'omphacine que dans l'huile classique.
- acide oléique :
- 81,5 g/100 g d'omphacine Picholine,
   contre 78,6 g/100 g d'huile classique Picholine.
- 77,4 g/100 g d'omphacine Lucques,
   contre 75,4 g/100 g d'huile classique Lucques.
   Soit une teneur plus élevée d'acide oléique dans l'omphacine que dans l'huile classique.

- biophénols totaux :
• 225 mg/kg d'omphacine Picholine (dont 221 mg/kg sous forme complexe),
contre 160 mg/kg d'huile classique Picholine (dont 134 mg/kg sous forme complexe).
• 211 mg/kg d'omphacine Lucques (dont 205 mg/kg sous forme complexe),
contre 123 mg/kg d'huile classique Lucques (dont 100,4 mg/kg sous forme complexe).
Soit une teneur plus élevée en biophénols totaux dans l'omphacine que dans l'huile classique.

| -rendement en jus et huile : | olives précoces | olives matures |
|---|---|---|
| huile | 5-9 % | 12-25 % |
| margines | 95-91 % | 88-75 % |

### COMPOSITION 1 : Jus intégral d'olive stabilisé

- Jus d'olives matures 100 g (Picholine)
- additifs : gomme xanthane 0,4 g
   ascorbate de sodium 0,3 g
   sorbate de potassium 0,5 g

Les ingrédients sont mélangés en évitant l'incorporation d'air, à froid.

La composition est éventuellement soumise à un traitement UHT et conditionnée dans un environnement stérile.

### COMPOSITION 2 : Jus intégral d'olive stabilisé

- Jus d'olives avant maturité 100 g (Picholine)
- additifs : gomme guar 0,5 g
   ascorbate de sodium 0,4 g
   sorbate de potassium 0,25 g
   benzoate de sodium 0,25 g

Même mode opératoire que pour la composition 1

La composition selon la présente invention peut se présenter sous diverses formes de produits finis, l'actif, c'est à dire le suc intégral, pouvant être associé à une multitude de composés eux-mêmes neutres ou dotés de propriétés avantageuses (huile, eau,...).

La fabrication à froid est évidemment privilégiée car elle permet de préserver toutes les propriétés et vertus du suc intégral. Elle peut être obtenue par émulgation des ingrédients ad hoc.

Sous forme d'assaisonnement ou de vinaigrette, le jus intégral d'olives matures (20 % en poids) peut être associé à de l'acide citrique, des aromates, du sel et du poivre. Une recette allégée pourra comprendre 7 % en poids de jus intégral d'olives vertes en lieu et place des 20 % de jus intégral d'olives matures.

Des laits cosmétiques à base de jus intégral d'olives vertes, des crèmes de jour à base de jus intégral d'olives matures ou des crèmes de nuit à base de jus intégral d'olives matures comprenant 10 % en poids d'huile d'olives vierge peuvent être obtenus ainsi que toutes présentations destinées à l'usage corporel ou capillaire formulées à base de jus intégral d'olives vertes ou matures : en particulier produits de bain, huiles corporelles, cataplasme ou emplâtres ou produits de soin capillaire. Ces produits sont formulés selon les règles que l'Homme de l'art sait définir en y incorporant notamment divers conservateurs (alcool benzylique, DHA,...), des émulgateurs (olivem, oléate, stéarate, eumulgen VL75, ...), des huiles essentielles d'agrumes par exemple ainsi que des colorants (Ti02) dans des proportions que l'Homme de l'art saura adapter selon les applications envisagées.

## Revendications

1. Procédé de préparation d'un jus intégral d'olive ***caractérisé en ce qu'***il comprend les étapes suivantes :
a) sélectionner des olives en fonction de leur maturité,
b) broyer et malaxer les olives choisies sous forme de drupes,
c) presser la pâte obtenue à l'étape précédente,
d) séparer le jus obtenu pour le dissocier des éléments solides,
e) recueillir le jus intégral d'olive sous forme d'une émulsion gélifiée stable.

2. Procédé selon la revendication 1, ***caractérisé en ce qu'***il comprend l'addition immédiatement après l'étape e) d'agents viscosants et éventuellement émulgateurs ou un mélange de ceux-ci.

3. Procédé selon la revendication 1 ou 2, ***caractérisé en ce qu'***il comprend à l'étape a) la sélection d'olives majoritairement récoltées à un stade précoce de maturité.

4. Procédé selon la revendication 3, ***caractérisé en ce qu'***il comprend à l'étape a) la sélection d'olives uniquement récoltées à un stade précoce de maturité.

5. Procédé selon la revendication 1 ou 2, ***caractérisé en ce qu'*il** comprend à l'étape a) la sélection d'olives majoritairement récoltées à un stade de maturité avancé.

6. Procédé selon la revendication 5, ***caractérisé en ce qu'*il** comprend à l'étape a) la sélection d'olives uniquement récoltées à un stade de maturité avancé.

7. Composition à base de jus intégral d'olives obtenu selon l'une quelconque des revendications 1 à 6, ***caractérisée en ce qu***'elle comprend un mélange de phase huileuse et de phase aqueuse d'olive.

8. Composition selon la revendication 7, ***caractérisée en** ce **qu***'elle comprend 5 à 25 % en poids de ladite composition de phase huileuse et 95 à 75 % en poids de ladite composition de phase aqueuse.

9. Composition selon la revendication 7 ou 8, ***caractérisée en ce qu***'elle comprend en outre des additifs choisis parmi les gélifiants, les antioxydants hydrosolubles, conservateurs, chélateurs ou un mélange de ceux-ci.

10. Utilisation d'une composition selon l'une quelconque des revendications 7 à 9 dans le domaine de la cosmétique et/ou de la diététique.

11. Utilisation selon la revendication 10, ***caractérisée en ce qu***'elle est réalisée dans le domaine de la cosmétique dans le cas d'une composition élaborée avec une majorité d'olives récoltées à un stade précoce de maturité.

12. Utilisation selon la revendication 11 pour la préparation d'une composition qui permet une action anti-radicalaire, émolliente et adoucissante.

13. Utilisation selon la revendication 10, ***caractérisée en ce qu***'elle est réalisée dans le domaine de la diététique dans le cas d'une composition élaborée avec une majorité d'olives récoltées à un stade de maturité avancé.

14. Utilisation selon la revendication 13 pour la préparation d'une composition qui permet une action anti-radicalaire, de prévention des affections cardio-vasculaires et des lésions tumorales.

## Patentansprüche

1. Verfahren zur Herstellung eines vollinhaltlichen Olivensaftes,
**dadurch gekennzeichnet ,**
**dass** es die folgenden Schritte aufweist:
a) Auswählen von Oliven als Funktion ihrer Reife,
b) Zerdrücken und Durchkneten der ausgewählten Oliven in Form von Steinfrüchten,
c) Auspressen der erhaltenen Paste im folgenden Schritt,
d) Trennen des erhaltenen Saftes, um ihn in feste Bestandteile zu entmischen,
e) Auffangen des vollinhaltlichen Olivensaftes in Form einer gelierten stabilen Emulsion.

2. Verfahren nach Anspruch 1,
**dadurch gekennzeichnet,**
**dass** es nach dem Schritt e) die sofortige Zugabe von Viskosemitteln und eventuell von Emulgatoren oder einer Mischung davon enthält.

3. Verfahren nach Anspruch 1 oder 2,
**dadurch gekennzeichnet,**
**dass** es in Schritt a) die Auswahl von Oliven enthält, die mehrheitlich in einem Frühstadium der Reife geerntet wurden.

4. Verfahren nach Anspruch 3,
**dadurch gekennzeichnet,**
**dass** es in Schritt a) die Auswahl von Oliven enthält, die einheitlich in einem Frühstadium der Reife geerntet wurden.

5. Verfahren nach Anspruch 1 oder 2,
**dadurch gekennzeichnet,**
**dass** es in Schritt a) die Auswahl von Oliven enthält, die mehrheitlich in einem fortgeschrittenen Reifestadium geerntet wurden.

6. Verfahren nach Anspruch 5,
**dadurch gekennzeichnet,**
**dass** es in Schritt a) die Auswahl von Oliven enthält, die einheitlich in einem fortgeschrittenen Reifestadium geerntet wurden.

7. Zusammensetzung auf Grundlage von vollinhaltlichem Olivensaft erhalten nach einem der Ansprüche 1 bis 6,
**dadurch gekennzeichnet,**
**dass** sie eine Mischung aus einer öligen Phase und einer wässrigen Phase der Olive enthält.

8. Zusammensetzung nach Anspruch 7,
**dadurch gekennzeichnet,**
**dass** sie 5 Gew.-% bis 25 Gew.-% der Zusammensetzung der öligen Phase und 95 Gew.-% bis 75 Gew.-% der Zusammensetzung der wässrigen Phase enthält.

9. Zusammensetzung nach Anspruch 7 oder 8,
**dadurch gekennzeichnet,**
**dass** sie zudem Zusätze enthält, die ausgewählt sind aus Gelierungsmitteln, wasserlöslichen Antioxidantien, Konservierungsmitteln, Chelatbildnern oder einer Mischung davon.

10. Verwendung einer Zusammensetzung nach einem der Ansprüche 7 bis 9 auf dem Gebiet der Kosmetik und/oder der Diätetik.

11. Verwendung nach Anspruch 10,
**dadurch gekennzeichnet,**
**dass** sie auf dem Gebiet der Kosmetik in dem Fall einer Zusammensetzung, die mit einer Mehrheit von in einem Frühstadium der Reife geernteten Oliven hergestellt ist, realisiert wird.

12. Verwendung nach Anspruch 11 für die Herstellung einer Zusammensetzung, welche eine antiradikalische, beruhigende und reizlindernde Wirkung erlaubt.

13. Verwendung nach Anspruch 10,
**dadurch gekennzeichnet,**
**dass** sie auf dem Gebiet der Diätetik in dem Fall einer Zusammensetzung, die mit einer Mehrheit von in einem fortgeschrittenen Reifestadium geernteten Oliven hergestellt ist, realisiert wird.

14. Verwendung nach Anspruch 13 für die Herstellung einer Zusammensetzung, die eine antiradikalische Wirkung erlaubt, zum Verhindern von kardiovaskulären Leiden und von Tumorverletzungen.

## Claims

1. Process for preparing integral olive liquor ***characterised in that it*** consists of the following stages:
a) selecting the olives according to their maturity,
b) grinding and mixing the olives selected in the form of drupes,
c) pressing the paste obtained in the previous phase,
d) separating the liquor obtained in order to separate it from the solid parts,
e) collecting the integral olive liquor in the form of a stable, gelified emulsion.

2. Process according to claim 1, ***characterised in that it*** comprises the addition immediately after stage e) of viscosing agents which may also be emulsifiers or a mixture of these.

3. Process according to claim 1 or 2, ***characterised in that*** at stage a) it includes the selection of olives mainly gathered at an early stage of maturity.

4. Process according to claim 3, ***characterised in that*** at stage a) it includes the selection of olives only gathered at an early stage of maturity.

5. Process according to claim 1 or 2, ***characterised in that*** at stage a) it includes the selection of olives mainly gathered at an advanced stage of maturity.

6. Process according to claim 5, ***characterised in that*** at stage a) it includes the selection of olives only gathered at an advanced stage of maturity.

7. Composition based on integral olive liquor obtained using any of the claims 1 to 6, ***characterised in that*** it comprises a mixture of the oily phase and the aqueous phase of olive.

8. Composition according to claim 7, ***characterised in that*** the invention preferably consists of 5 to 25% by weight of said composition of the oily phase with 95 to 75% by weight of said composition of the aqueous phase.

9. Composition according to claim 7 or 8, ***characterised in that*** it includes additives selected from among the gelling agents, hydrosoluble antioxidants, preservatives, chelators or a mixture of these.

10. Use of a composition according to any of claims 7 to 9 in the field of cosmetics and/or nutrition.

11. Use according to claim 10, ***characterised in that*** it is carried out in the field of cosmetics in the case of a composition produced mainly with olives gathered aAAt an early stage of maturity.

12. Use according to claim 11 for the preparation of a composition which is able to offer an anti-radical, emollient and softening action.

13. Use according to claim 10, ***characterised in that*** it is carried out in the field of nutrition in the case of a composition produced mainly with olives gathered at an advanced stage of maturity.

14. Use according to claim 13 for the preparation of a composition which is able to offer an antiradical action, to prevent cardiovascular disease and tumoral lesions.
